# DEMANDE DE BREVET EUROPEEN

(11) **EP 3 459 552 A1**
(43) Date de publication de la demande: **27.03.2019**
(21) Numéro de dépôt: 18203891.9
(22) Date de dépôt: 09.10.2015
(51) Int. Cl.: A61K 35/16, B01D 15/38

(54) **PROCÉDÉ DE PREPARATION DE PLASMA UNIVERSEL**

(30) Priorité: 09.10.2014 FR 1459679
(62) Demande divisionnaire de: 15784303.8
(71) Demandeur: Laboratoire Français du Fractionnement et des Biotechnologies, 91940 Les Ulis (FR)
(72) Inventeur: CHTOUROU, Abdessatar, 78990 ELANCOURT (FR)
(74) Mandataire: IPAZ

(57) **Abrégé**

La présente invention concerne un plasma universel, issu d'un mélange de plasmas provenant des d'individus donneurs de groupes sanguins A, B, AB et/ou O et compatible avec tous les groupes sanguins et son procédé d'obtention.

## Description

### Domaine technique

La présente invention concerne un plasma universel, sécurisé viralement, appauvri en anticorps anti-A et anti-B, et compatible avec tous les groupes sanguins, ainsi que son procédé d'obtention.

### Arrière-plan technologique

Le "plasma sanguin" ou "plasma" est le composant liquide du sang, dans lequel les cellules sanguines sont en suspension. Le plasma est susceptible d'être séparé du sang total par centrifugation ou filtration à travers une membrane. Le plasma sanguin est constitué essentiellement d'eau, de protéines plasmatiques (principalement l'albumine et des anticorps) et des facteurs de coagulation dont le fibrinogène.
Le plasma sanguin est utilisé en thérapeutique pour le traitement de coagulopathies graves avec effondrement de tous les facteurs de coagulation ainsi que pour le traitement d'hémorragies aiguës, avec déficit global des facteurs de coagulation.

Actuellement, le plasma thérapeutique généralement utilisé est du plasma frais congelé (PFC) issu de dons qualifiés biologiquement (tests immunohématologiques, recherche de virus VIH, VHC et VHB par amplification des acides nucléiques) et qui peut être sécurisé vis-à-vis des pathogènes par quarantaine ou traitement physico-chimique. Un tel plasma nécessite une conservation à une température inférieure ou égale à -25°C, ce qui implique que son administration ne peut s'effectuer qu'après décongélation dans des conditions spécifiques. De plus, ce type de plasma ne peut être conservé plus d'un an, ce qui génère des pertes conséquentes. Enfin, ce plasma thérapeutique doit être utilisé avec précaution puisque son administration doit respecter les règles de délivrance basées sur les compatibilités ABO des receveurs. Au-delà de l'utilisation de plasmas compatibles, la transfusion de plasma iso-groupe doit donc être privilégiée afin d'éviter tout risque d'hémolyse post-transfusionnelle par incompatibilité ABO.

Du fait des restrictions liées à son utilisation et à sa conservation et donc sa disponibilité, un tel plasma frais congelé s'avère inadapté à une application dans des conditions d'urgence, notamment dans le milieu hospitalier ou sur le terrain hors milieu hospitalier (lieux d'accidents ou de catastrophes, zones difficiles d'accès, zones à accès restreint ou inexistant aux chaînes du froid, terrains d'opérations militaires).

Il existe donc un besoin pour un plasma répondant aux contraintes du milieu hospitalier et non hospitalier, susceptible d'être conservé à température ambiante, exempt de toute forme de contamination bactérienne, virale ou parasitaire et compatible avec n'importe quel receveur, indépendamment de son groupe sanguin ABO.

La demande WO2012/022914 décrit un procédé d'obtention d'un plasma lyophilisé, issu d'un mélange de plasma de donneurs sélectionnés et appartenant exclusivement au groupe sanguin AB ou aux groupes sanguins A ou B. Le procédé décrit dans la demande WO2012/022914 ne peut être mis en oeuvre avec des donneurs appartenant au groupe sanguin O.

La demande US20110008459 décrit un plasma universel obtenu en mélangeant les plasmas de donneurs de groupes sanguins A et B et éventuellement AB à l'exclusion de tout plasma provenant de donneurs de groupe sanguin O, dans lequel le titre en anticorps anti-A et anti-B libres est inférieur à 16 pour les immunoglobulines M (IgM) et inférieure à 64 pour les immunoglobulines G (IgG).

### Résumé de l'invention

Les inventeurs ont développé un procédé de préparation d'un plasma universel dont les caractéristiques répondent à des exigences de conservation et d'utilisation encore non satisfaites et ceci, quel que soit le groupe sanguin auquel appartient ce plasma.

Ainsi l'invention concerne un procédé de préparation d'un plasma universel, comprenant les étapes suivantes :
a) Mélange de plasma unitaire non-universel obtenu à partir d'un échantillon de donneurs,
b) Elimination des anticorps anti-A et anti-B présents dans le plasma par chromatographie d'immunoaffinité ou par déplétion en batch, et
c) éventuellement la lyophilisation ou l'atomisation du plasma universel issu de l'étape b).

Le procédé de l'invention permet *in fine* l'obtention d'un « plasma universel » pouvant être administré à tout patient quel que soit son groupe sanguin et sans détermination au préalable du groupe sanguin du patient. Le plasma universel selon l'invention répond aux exigences réglementaires auxquelles sont soumis les plasmas actuellement utilisés en thérapeutique, en particulier les concentrations en facteurs de coagulation sont satisfaisantes (par exemple la concentration en facteur VIII est supérieure ou égale à 0,5 UI/mL, la concentration en facteur V est supérieure ou égale à 0,7 UI/mL, la concentration en facteur XI est supérieure ou égale à 0,5 UI/mL et la concentration en fibrinogène est supérieure ou égale à 2g/L) et il n'y a pas d'activation des facteurs de la coagulation.

Dans un mode de réalisation particulier, l'étape b) est réalisée au moyen d'une chromatographie d'immunoaffinité ou d'une déplétion en batch sur un support dont la matrice est greffée de groupes oligosaccharidiques antigéniquement similaires aux groupes sanguins A et/ou B, par exemple selon les techniques décrites dans la demande WO2007/077365 ou utilisant une matrice telle que décrite dans les demandes FR 13 56635 ou FR 13 56636.

Dans un mode de réalisation particulier, lesdits donneurs appartiennent au groupe sanguin sélectionné parmi le groupe sanguin A, le groupe sanguin B, le groupe sanguin AB et/ou le groupe sanguin O.

Dans un mode de réalisation particulier, le procédé de préparation d'un plasma universel peut en outre comprendre une étape de lyophilisation, de cryodessiccation ou d'atomisation à l'issue de l'étape b), permettant l'obtention d'un « plasma universel lyophilisé », d'un « plasma universel cryodesséché » ou d'un « plasma universel atomisé », grâce à une étape de lyophilisation, de cryodessiccation ou d'atomisation appropriée.

Dans un mode de réalisation particulier, le plasma universel issu de l'étape b) présente une teneur en anticorps anti-A et/ou une teneur en anticorps anti-B contenue dans le plasma universel conforme à un résultat négatif à la dilution 1/64 du test de Coombs, réalisé selon la méthode 2.6.20 de la Pharmacopée européenne 07/2011:20620.

Dans un mode de réalisation particulier, le procédé de préparation d'un plasma universel peut comprendre en outre une étape de sécurisation biologique, par inactivation virale et/ ou ultrafiltration en amont de l'étape a).

La présente invention concerne également un plasma universel, de préférence un plasma universel dans lequel la teneur en anticorps anti-A et la teneur en anticorps anti-B est conforme à un résultat négatif au test de Coombs à la dilution 1/64 du test de Coombs, réalisé selon la méthode 2.6.20 de la Pharmacopée européenne 07/2011:20620.

La présente invention concerne également l'utilisation d'un tel plasma universel pour le traitement de patients :
- souffrant d'hémorragies sévères, notamment d'origine traumatique, en remplacement de multiples facteurs de la coagulation, en situation d'urgence lorsqu'un concentré de facteurs de coagulation n'est pas disponible,
- souffrant d'hémorragies traumatiques ou spontanées sous anticoagulants oraux (ACO),
- souffrant de déficit sévère en vitamine K, ou
- atteints de purpura thrombotique thrombocytopénique.

D'autres caractéristiques et avantages de la présente invention apparaîtront à la lecture de la description détaillée qui suit et du mode de réalisation préféré de l'invention, donné à titre d'exemple.

### Description détaillée

Par "plasma unitaire non universel", on entend du plasma recueilli à partir d'un seul individu donneur humain, quel que soit son groupe sanguin. Ce plasma unitaire peut être préparé à partir du sang total ou être recueilli par aphérèse. Ce plasma unitaire non universel est un plasma humain. Préférentiellement, dans le cadre de cette invention, les plasmas unitaires non universels constituant le mélange de plasmas de l'étape a) du procédé de l'invention sont recueillis par aphérèse. Préférentiellement, les plasmas unitaires non universels constituant le mélange de plasmas de l'étape a) répondent aux mêmes exigences réglementaires auxquelles sont soumis les plasmas actuellement utilisés en thérapeutique ou pour le fractionnement. Ils respectent donc les exigences en vigueur, notamment en termes de taux de facteurs d'hémostase. Les individus donneurs doivent donc respecter les critères réglementaires d'éligibilité au don du plasma. Préférentiellement, les plasmas unitaires non universels sont obtenus à partir de donneurs masculins ou exempts d'anticorps anti-HLA. En outre, dans le contexte de la présente invention, les donneurs doivent présenter un bilan d'hémostase normal et caractérisé par un taux de facteur VIII au moins égal à 0,9 UI/mL.

Par "individu donneur humain", on entend un individu humain susceptible de faire don (gratuit ou rémunéré) de son sang ou des composants sanguins.

Par "mélange de plasmas" ou "pool de plasmas ", on entend un mélange de plasmas humains unitaires non universel. Dans le contexte de l'invention, le « pool de plasmas » peut être un pool de plasmas thérapeutiques, directement utilisable en thérapie ou un pool de plasmas pour fractionnement. Selon l'invention, le pool de plasmas est avantageusement déleucocyté pour sécuriser le plasma vis-à-vis des risques de transmission du prion ou d'autres agents infectieux. Avantageusement, le plasma déleucocyté selon l'invention possède une teneur limite en leucocytes résiduels ≤1,0 x 10⁶/litre. Préférentiellement, un « mélange de plasmas » ou « pool de plasmas » correspond à un mélange d'au moins 2 plasmas humains unitaires non universels, préférentiellement d'au moins 5 plasmas humains unitaires non universels, encore plus préférentiellement d'au moins 10 plasmas humains unitaires non universels.

Dans un mode de réalisation de l'invention, un « mélange de plasmas » ou « pool de plasmas » correspond à un mélange d'au moins 100 plasmas humains unitaires non universels différents. Dans un autre mode de réalisation particulier de l'invention, un « mélange de plasmas » ou « pool de plasmas » correspond à un mélange d'au moins 1 500 plasmas humains unitaires non universels différents.

Dans un mode de réalisation particulier de l'invention, un « mélange de plasmas » ou « pool de plasmas » correspond à un mélange d'au moins 2 000 plasmas humains unitaires non universels différents. Dans un autre mode de réalisation particulier de l'invention, un « mélange de plasmas » ou « pool de plasmas » correspond à un mélange d'au moins 10 000 plasmas humains unitaires non universels différents.

Dans un autre mode de réalisation particulier de l'invention, le nombre de plasmas unitaires non universels utilisés pour constituer un « mélange de plasmas » ou « pool de plasmas » est avantageusement ajusté pour garantir la sécurité biologique. Ainsi, l'ajout d'étapes supplémentaires de sécurisation biologique permet d'augmenter le nombre de plasmas unitaires non universels tout en minimisant le risque biologique.

Par "sang total", on entend l'ensemble des composés et des cellules constituant le sang.

Par "aphérèse", on entend une technique de prélèvement, chez un donneur, de certains composants sanguins. Les composants que l'on souhaite prélever sont séparés par centrifugation et stockés, tandis que les composants non prélevés sont réinjectés au donneur.

Dans un mode de réalisation particulier, le mélange de plasmas de l'étape a) du procédé selon l'invention est obtenu à partir des plasmas unitaires non universels d'au moins 2 donneurs différents, d'au moins 5 donneurs différents, préférentiellement d'au moins 10 individus donneurs humains différents. Dans un mode de réalisation particulier, le mélange de plasmas unitaires non universels de l'étape a) du procédé selon l'invention est obtenu à partir de 100 individus donneurs humains différents. Dans un autre mode de réalisation particulier, le mélange de plasmas unitaires non universels de l'étape a) du procédé selon l'invention est obtenu à partir de 1 500 individus donneurs humains différents.

Une telle limitation quant au nombre d'individus donneurs humains permet de réduire considérablement le risque infectieux résiduel tout en bénéficiant des avantages du mélange : immunogénicité réduite, effet bénéfique sur le risque infectieux du fait de la dilution ou de la neutralisation des agents pathogènes, et obtention d'un plasma universel pour le groupage sanguin. Il permet en outre une traçabilité simplifiée des individus donneurs.

Dans un autre mode de réalisation particulier, le mélange de plasmas unitaires non universels de l'étape a) du procédé selon l'invention est obtenu à partir d'au moins 2 000 individus donneurs humains, préférentiellement à partir d'au moins 5 000, encore plus préférentiellement à partir d'au moins 10 000 individus donneurs humains différents. Afin de ne pas limiter le nombre d'individus donneurs humains, des mesures adéquates supplémentaires peuvent être mises en place pour caractériser et sécuriser les plasmas unitaires non universels: écartement des donneurs à risque, quarantaine, recherche du génome du parvovirus B19 ou du virus de l'hépatite A, de l'hépatite B ou du VIH par amplification nucléique, recherche d'anticorps anti-virus de l'hépatite C, étape supplémentaire d'élimination ou d'inactivation virale, ou toute autre mesure de sécurisation biologique adaptée.

Préférentiellement, lesdits individus donneurs humains appartiennent aux groupes sanguins A, B, AB ou O, ce qui permet de ne pas exclure de catégories de donneurs sur la base de leur groupe sanguin, et donc, d'augmenter les volumes de plasma source utilisables selon l'invention. Par "individu donneur humain appartenant au groupe sanguin A, B, AB ou O", on entend un individu donneur possédant respectivement le phénotype A, B, AB ou O. L'appartenance à un groupe sanguin particulier n'a aucune influence sur le procédé de l'invention. Aucune sélection préalable des plasmas unitaires non universels n'est effectuée en amont dudit procédé.

La Demanderesse a trouvé, de façon surprenante et inattendue, que le procédé selon l'invention permettait l'obtention d'un plasma universel à partir de plasmas unitaires non universels, et ce, quel que soit le groupe sanguin auquel appartiennent les individus donneurs. En d'autres termes, le procédé de l'invention permet l'obtention d'un plasma universel à partir de plasmas unitaires non universels provenant d'un échantillon d'individus donneurs appartenant au groupe sanguin A, B, AB ou O. Dans un mode de réalisation particulier, le mélange de plasmas unitaires non universels de l'étape a) du procédé selon l'invention provient avantageusement d'individus donneurs appartenant aux groupes sanguins A, B, AB et O.

Par "groupe sanguin", on entend une classification de sang reposant sur la présence ou l'absence de substances antigéniques sur la surface des hématies. Ces substances antigéniques définissent le système ABO. L'antigène A correspond à la présence d'une N-acétyl-galactosamine greffée en position α₁₋₃de l'épitope (chaîne H). L'antigène B correspond à la présence d'un galactose greffé en position α₁₋₃ de l'épitope (chaîne H). Le système ABO dicte les règles de compatibilité de la transfusion sanguine. Le non-respect de ces règles peut entraîner un accident hémolytique chez l'individu transfusé. Comme le plasma contient des anticorps en fonction du groupe dans le système ABO, les globules rouges du receveur ne doivent pas présenter les antigènes correspondants. Ainsi, il convient de ne pas administrer un plasma comprenant des anticorps anti-A à un patient appartenant au groupe sanguin A, et vice versa.

Par « anticorps anti-A » ou « anticorps anti-groupe sanguin A », on entend tout anticorps reconnaissant les antigènes du groupe sanguin A.

Par « anticorps anti-B » ou « anticorps anti-groupe sanguin B », on entend tout anticorps reconnaissant les antigènes du groupe sanguin B.

Dans un mode de réalisation particulier de l'invention, les anticorps anti-A et anticorps anti-B consistent en des immunoglobulines reconnaissant les antigènes du groupe sanguin A et/ou B, en particulier des immunoglobulines G ou M.

Dans un autre mode de réalisation particulier de l'invention, les anticorps anti-A et anticorps anti-B consistent en des immunoglobulines G ou M reconnaissant les antigènes du groupe sanguin A et/ou B, en particulier des hémolysines ou agglutinines. Les anticorps anti-A et anti-B de type hémolysine sont des immunoglobulines G susceptibles d'être présentes dans le plasma et d'entraîner la lyse des hématies.

Par « plasma universel », on entend un plasma utilisable pour tout individu receveur, indépendamment de son groupe sanguin. Le plasma universel permet avantageusement de s'affranchir de la contrainte de compatibilité ABO ou d'iso-groupe ABO entre les individus donneurs et receveurs. Il est ainsi possible de ne produire et conserver qu'un seul type de plasma, universel, au lieu des 4 types de plasma (A, B, AB et O) actuellement nécessaires pour satisfaire à la compatibilité ABO. Le plasma universel évite ainsi les gaspillages de plasma périmé dû aux mauvaises anticipations des besoins selon les groupes sanguins des patients. Un tel plasma universel permet avantageusement de réduire l'espace dédié au stockage du plasma, et supprime également les risques de transfusion non compatible accidentelle. Le « plasma universel » consiste notamment en un plasma appauvri en tout anticorps dirigé contre les antigènes du groupe sanguin A et/ou du groupe sanguin B, en particulier appauvri en anticorps anti-A et/ou en anticorps anti-B.

Avantageusement, le plasma obtenu à l'issu de l'étape b) du procédé de l'invention ne contient pas d'anticorps anti-A ou anti-B ou contient un taux résiduel d'anticorps anti-A et/ou anti-B inférieur conforme à un résultat négatif à la dilution 1/64 du test de Coombs et est qualifié de "plasma universel ".

Dans un mode de réalisation particulier de l'invention, le plasma universel présente avantageusement un résultat négatif au test de Coombs à la dilution 1/32, encore plus avantageusement à la dilution 1/16, de manière encore plus avantageuse à la dilution 1/8 ou inférieure.

Par « test de Coombs » on entend le test de Coombs direct ou indirect tel que décrit au paragraphe 2.6.20 de la Pharmacopée Européenne 01/2005:20620 ou au chapitre 2.6.20 de la Pharmacopée Européenne 01/2008:20620, ou de la Pharmacopée Européenne 07/2011 :20620. Le test de Coombs indirect consiste en une méthode indirecte utilisant une antiglobuline telle que décrite par exemple au chapitre 2.6.20 de la Pharmacopée Européenne 01/2005:20620 ou de la Pharmacopée Européenne 07/2011 : 20620. Le test de Coombs direct consiste en une méthode d'hémagglutination directe, utilisant des cellules traitées à la papaïne, comme méthode de référence pour la recherche des anticorps anti-A et anti-B avec utilisation de 3 standards (un témoin positif, un témoin négatif et une préparation de référence pour essai limite) telle que décrite par exemple au chapitre 2.6.20 de la Pharmacopée Européenne 01/2008:20620 ou de la Pharmacopée Européenne 07/2011:20620.

L'étape b) dudit procédé concerne l'élimination des anticorps anti-A et anti-B présents dans le plasma par chromatographie d'immunoaffinité ou par déplétion en batch. Cette étape est essentielle pour obtenir un plasma universel, directement administrable au receveur quel que soit le groupe sanguin ABO de ce dernier.

Le plasma issu de l'étape a) est soumis à une étape chromatographique d'immunoaffinité ou de déplétion en batch sur un support greffé de groupes antigéniquement similaires aux groupes sanguins A et/ou B, de préférence sur une colonne remplie d'un tel support. De préférence, le support chromatographique ou de déplétion en batch est constitué d'une matrice en polymère sur laquelle sont greffés des espaceurs ou des bras de couplage, étant à leur tour greffés, avec des oligosaccharides représentant avantageusement des trisaccharides correspondants aux épitopes des groupes sanguins A et B.

On entend par « déplétion en batch » une méthode de purification par affinité à l'aide d'un support greffé de ligands spécifiques, les fractions d'élution et de lavage étant séparées du tel support après incubation avec les molécules d'intérêt par centrifugation afin de récupérer ledit support.

La matrice de chromatographie d'immunoaffinité ou de déplétion en batch, comprend des particules de polymère sur lesquelles au moins un oligosaccharide correspondant à un épitope de groupe sanguin A et/ou de groupe B est greffé, ledit oligosaccharide étant greffé auxdites particules via un espaceur, caractérisée en ce que ledit espaceur présente la formule (I) -NH-R₁-CO-NH-R₂-, dans laquelle R₁ est un groupe alkyle en C₄-C₆, R₂ est un groupe alkyle en C₃-C₈, et ledit espaceur est lié par sa fonction amine à la particule de polymère.

Dans un mode de réalisation particulier, la matrice comprend (i) des particules de polymère sur lesquelles au moins un oligosaccharide correspondant à un épitope de groupe sanguin A est greffé, et/ou (ii) des particules de polymère sur lesquelles au moins un oligosaccharide correspondant à un épitope de groupe sanguin B est greffé. Dans un mode de réalisation préféré, la matrice comprend (i) des particules de polymère sur lesquelles au moins un oligosaccharide correspondant à un épitope de groupe sanguin A est greffé, et (ii) des particules de polymère sur lesquelles au moins un oligosaccharide correspondant à un épitope de groupe sanguin B est greffé.

Dans un autre mode de réalisation, la matrice comprend des particules de polymère sur lesquelles à la fois au moins un oligosaccharide correspondant à un épitope de groupe sanguin A, et au moins un oligosaccharide correspondant à un épitope de groupe sanguin B, sont greffés.

Dans un mode de réalisation particulier, la matrice comprend un mélange de (i) particules de polymère sur lesquelles au moins un oligosaccharide correspondant à un épitope de groupe sanguin A est greffé, (ii) particules de polymère sur lesquelles au moins un oligosaccharide correspondant à un épitope de groupe sanguin B est greffé, et (iii) particules de polymère sur lesquelles à la fois au moins un oligosaccharide correspondant à un épitope de groupe sanguin A, et au moins un oligosaccharide correspondant à un épitope de groupe sanguin B, sont greffés.

En particulier, de très bons résultats sont obtenus en mettant en oeuvre un tel support dont les trisaccharides, correspondant à l'épitope du groupe sanguin A, présentent la structure N-acétylgalactosamine (GalNAc) - Galactose (Gal) - Fucose (Fuc), et ceux correspondant à l'épitope du groupe sanguin B, présentent la structure Galactose-Galactose-Fucose (Gal-Gal-Fuc).

Dans un mode de réalisation préféré, sont mélangées des billes de cellulose réticulée sur lesquelles sont greffés des ligands qui sont des trisaccharides correspondant à un épitope du groupe sanguin A (N-acétylgalactosamine (GalNAc)-Galactose(Gal)- Fucose), et des billes de cellulose réticulée sur lesquelles sont greffés des ligands qui sont des trisaccharides correspondant à un épitope du groupe sanguin B (Galactose-Galactose-Fucose).

Les trisaccharides sont greffés aux billes par un espaceur de formule : NH-C₅H₁₀-CO-NH-C₃H₆.

### Le support :

La matrice de l'invention comprend un support à base de particules de polymère, et est de préférence sous forme d'un gel ou d'une résine.

Ces particules de polymère sont de préférence de forme sphérique ou oblongue, il peut s'agir notamment de billes. Ces particules de polymère ont généralement une taille moyenne d'environ 0,1µm à environ 1000µm, de préférence d'environ 20 à environ 500µm, de préférence encore d'environ 50 à environ 200µm, de préférence encore d'environ 70µm à environ 120µm de diamètre. De préférence ces particules sont poreuses.

Le polymère peut être naturel ou non-naturel, organique ou inorganique, réticulé ou non réticulé. Le polymère est de préférence un polymère organique, de préférence réticulé.

Dans un mode de réalisation préféré, le polymère est de la cellulose, et les particules sont de préférence des billes de cellulose poreuses. De préférence encore, il s'agit de cellulose réticulée.

D'autres types de polymères possibles incluent agarose, dextran, polyacrylates, polystyrène, polyacrylamide, polyméthacrylamide, des copolymères de styrène et divinylbenzène, ou des mélanges de ces polymères.

### Les ligands :

Les ligands portés par le support selon l'invention sont des oligosaccharides représentant les antigènes des groupes sanguins A et/ou B, qui sont naturellement des oses.

Plus précisément l'oligosaccharide correspondant à un épitope de groupe sanguin A et/ou l'oligosaccharide correspondant à un épitope de groupe sanguin B porté(s) par la matrice selon l'invention sont typiquement des trisaccharides. Le terme «oligosaccharides correspondants à un épitope de groupe sanguin» se réfère à des motifs identiques ou similaires, d'un point de vue antigénique, aux déterminants antigéniques reconnus par les anticorps anti-A et anti-B, respectivement. Les ligands portés par le support selon l'invention sont donc spécifiques des anticorps anti-A ou anti-B.

De préférence l'oligosaccharide correspondant à un épitope de groupe sanguin A, utilisé comme ligand dans l'invention, est un trisaccharide N-acétylgalactosamine (GalNAc)- Galactose(Gal)-Fucose, plus précisément N-acétylGalα₁₋₃(FuCα₁₋₂)Gal, l'espaceur est lié par l'atome d'oxygène lié de préférence au carbone en position 1 du galactose.

L'oligosaccharide correspondant à un épitope de groupe sanguin B, utilisé comme ligand dans l'invention, est de préférence un oligosaccharide Galactose-Galactose-Fucose, plus précisément Galα₁₋₃(Fucα₁₋₂)Gal, l'espaceur est lié par l'atome d'oxygène lié de préférence au carbone en position 1 du galactose.

De manière avantageuse, la densité de ligands, c'est-à-dire la quantité de ligands (à savoir d'oligosaccharides correspondant à un épitope de groupe sanguin A ou B) greffés, par volume de matrice sous forme de gel, peut être comprise entre environ 0,2 et environ 0,7 mg/ml de matrice, de préférence encore entre environ 0,3 et environ 0,4mg/ml de matrice. De préférence la densité d'oligosaccharides est d'environ 0,3mg/ml de matrice.

L'espaceur, possède la formule (I) -NH-R₁-CO-NH-R₂-,dans laquelle :
- R₁ est un groupe alkyle en C₄-C₆, linéaire ou ramifié,
- R₂ est un groupe alkyle en C₃-C₈, linéaire ou ramifié,
ledit espaceur étant lié par sa fonction amine (en gras ci-dessus) à la particule de polymère.

Dans un mode de réalisation particulier, R₁ représente un groupe alkyle linéaire, de préférence, R₁ est un groupe alkyle en C₅ et R₂ représente un groupe alkyle linéaire, de préférence, R₂ est un groupe alkyle en C₃.

Une particule de polymère peut porter un ou plusieurs espaceurs.

L'espaceur permet de réduire l'encombrement stérique et d'augmenter l'accessibilité du ligand vis-à-vis des anticorps anti-A et anti-B à lier.

L'espaceur sert à immobiliser, sur une particule, soit un oligosaccharide correspondant à un épitope de groupe sanguin A, qui est de préférence un trisaccharide N-acétylGalα₁₋₃(FuCα₁₋₂)Gal tel que décrit ci-dessus, soit un oligosaccharide correspondant à un épitope de groupe sanguin B, qui est de préférence un trisaccharide Galα₁₋₃(FuCα₁₋₂)Gal tel que décrit ci-dessus.

Dans un mode de réalisation préféré, les ligands (de préférence des trisaccharides tels que décrits plus haut) sont greffés aux particules de polymère par un espaceur de formule : NH-C₅H₁₀-CO-NH-C₃H₆.

Un tel support chromatographique ou de déplétion en batch représente très avantageusement un gel ou résine telle que décrite dans les demandes WO2007/077365, FR 13 56635 ou FR 13 56636 ou un gel ou une résine disponible dans le commerce tels que GLYCOSORB® ABO (Glycorex Transplantation AS - Suède), la matrice d'affinité Sepharose 4B greffée de trisaccharides des groupes sanguins A et B (Dextra Laboratories Limited - Grande-Bretagne), ou toute autre matrice équivalente. Ce support chromatographique ou de déplétion en batch permet l'élimination simultanée des anticorps anti-A et anti-B en une seule étape de chromatographie d'immunoaffinité ou de déplétion en batch.

Dans un mode de réalisation préféré, les particules de polymère sur lesquelles au moins un oligosaccharide correspondant à un épitope de groupe sanguin A est greffé, et les particules de polymère sur lesquelles au moins un oligosaccharide correspondant à un épitope de groupe sanguin B est greffé, peuvent ensuite être mélangées, par exemple dans une proportion de 25/75 à 75/25 (v/v), de préférence environ 50/50 (v/v). Il est en effet possible d'ajuster la proportion des deux ligands dans la colonne à la population des donneurs selon la répartition des groupes sanguins de celle-ci.

Les particules de polymère sur lesquelles au moins un oligosaccharide correspondant à un épitope de groupe sanguin A est greffé, et les particules de polymère sur lesquelles au moins un oligosaccharide correspondant à un épitope de groupe sanguin B est greffé, peuvent également, le cas échéant, être mélangées à des particules de polymère portant à la fois au moins un oligosaccharide correspondant à un épitope de groupe sanguin A, et au moins un oligosaccharide correspondant à un épitope de groupe sanguin B.

Avantageusement, la matrice choisie n'entraine pas d'activation non souhaitée des facteurs de coagulation présents dans le plasma à traiter.

### Chromatographie d'immunoaffinité ou déplétion en batch:

La matrice telle que définie ici est utilisée dans une chromatographie d'immunoaffinité ou une déplétion en batch liant des anticorps anti- A et anti- B.

Selon la matrice utilisée, on obtiendra
- soit une matrice dite « anti-A » c'est-à-dire contenant uniquement des particules de polymère sur lesquelles sont greffés uniquement des oligosaccharides correspondant à un épitope de groupe sanguin A
- soit une matrice dite « anti-B » c'est-à-dire contenant uniquement des particules de polymère sur lesquelles sont greffés uniquement des oligosaccharides correspondant à un épitope de groupe sanguin B
- soit une matrice dite « anti-A anti-B » c'est-à-dire contenant à la fois des particules de polymère sur lesquelles sont greffés au moins un oligosaccharide correspondant à un épitope de groupe sanguin A et des particules de polymère sur lesquelles sont greffés au moins un oligosaccharide correspondant à un épitope de groupe sanguin B.

Dans un mode de réalisation particulier, les matrices présentent les formules suivantes :
support - espaceur - ligand (trisaccharide).

Dans un mode de réalisation particulier, les matrices présentent les formules suivantes :
Matrice « anti-A » :
   support - espaceur - trisaccharide correspondant à l'épitope du groupe sanguin A,
Matrice « anti-B » :
   support - espaceur - trisaccharide correspondant à l'épitope du groupe sanguin B,
Matrice « anti-A et anti-B » :
   support - espaceur - trisaccharide correspondant à l'épitope du groupe sanguin A et support - espaceur - trisaccharide correspondant à l'épitope du groupe sanguin B

Plus particulièrement :
Matrice « anti-A » :
   (particule de polymère)- NH-C₅H₁₀-CO-NH-C₃H₆-(N-acétylGalα₁₋₃(Fucα₁₋₂)Gal)
Matrice « anti-B » :
   (particule de polymère)- NH-C₅H₁₀-CO-NH-C₃H₆- (Galα₁₋₃((Fuc α₁₋₂)Gal)
Matrice « anti-A et anti-B » :
   (particule de polymère)- NH-C₅H₁₀-CO-NH-C₃H₆-(N-acétylGalα₁₋₃(Fucα₁₋₂)Gal) et (particule de polymère)- NH-C₅H₁₀-CO-NH-C₃H₆- (Galα₁₋₃((Fuc α₁₋₂)Gal)

La matrice peut être introduite dans une colonne de chromatographie. A l'échelle industrielle, la colonne peut contenir de 1 à 150 litres, avantageusement de 1 à 100 litres préférentiellement de 1 à 50 litres. Dans le cas d'une mise en oeuvre à l'échelle pilote, on peut utiliser des colonnes de 1 à 50 cm de hauteur, le diamètre est alors adapté à la hauteur de colonne utilisée. Le volume de matrice utilisé dans la colonne est ajusté en fonction du taux résiduel en anticorps anti-A et/ou anti-B souhaité par rapport au taux d'anticorps anti-A et/ou anti-B initial de la solution.

Les anticorps anti-A et/ou anti-B présents dans le plasma se fixent à la matrice, et le produit (plasma) non adsorbé est récupéré, appauvri en anticorps anti-A et/ou anti-B. Le plasma est versé à travers la matrice à une vitesse et dans des conditions qui permettent la liaison des anticorps avec les ligands portés par la matrice.

Les matrices sont adaptées à un usage industriel et peuvent être avantageusement ré-utilisées de manière répétée, sans dégradation. Leur régénération peut être réalisée par des procédures connues de l'homme du métier, par exemple par traitement par de la soude (NaOH), par exemple à 1 M.

Avantageusement, les étapes de régénération sont adaptées pour tenir compte de l'encrassement de la colonne dû au passage de plasma.

De préférence, le pH du plasma est ajusté à un pH supérieur ou égal à 5, avantageusement supérieur ou égal à 6, avantageusement à un pH d'environ 7,4 avant la chromatographie ou la déplétion en batch.

La charge de la colonne est adaptée au taux résiduel recherché d'anticorps anti-A et/ou anti-B. La spécificité d'une telle matrice ne nécessite pas un conditionnement préalable du plasma, c'est-à-dire que toute fraction ou plasma peut convenir.
La percolation du plasma ne fait pas intervenir de mécanisme d'élution. Par conséquent, le plasma est percolé à travers la colonne, éventuellement grâce à une pompe. Cette percolation permet la rétention des anticorps anti-A et anti-B. Le temps de contact entre les ligands et le plasma est supérieur ou égal à 30 secondes, avantageusement supérieur ou égal à une minute, préférentiellement supérieur ou égal à 2 minutes.

L'étape b) permet donc l'élimination des anticorps anti-A et B. De préférence, l'étape b) permet d'atteindre une teneur résiduelle en anticorps anti-A et anti-B telle que le résultat du test de Coombs est négatif à la dilution 1/64, permettant in fine l'obtention d'un plasma universel selon le procédé de l'invention qui pourra être utilisé sur n'importe quel receveur, indépendamment de son groupe sanguin.

Dans un mode de réalisation préféré, le plasma universel issu de l'étape b) présente une teneur en anticorps anti-A et une teneur en anticorps anti-B conforme à un résultat négatif au test de Coombs à la dilution 1/64. De préférence, la teneur en anticorps anti-A est telle que le résultat du test de Coombs est négatif à la dilution 1/32, de préférence négatif à la dilution 1/16, encore plus préférentiellement négatif à la dilution 1/8 ou inférieure. De préférence, la teneur en anticorps anti-B est telle que le résultat du test de Coombs est négatif à la dilution 1/32, de préférence négatif à la dilution 1/16, encore plus préférentiellement négatif à la dilution 1/8 ou inférieure.

Dans un mode de réalisation particulier, le plasma ainsi déplété en anticorps anti-A et anti-B peut ensuite être soumis à une étape de lyophilisation (étape c) ou de cryodessication ou d'atomisation. L'étape de lyophilisation est particulièrement délicate puisqu'il convient de ne pas compromettre les propriétés hémostatiques du mélange de plasmas sécurisés. Il convient alors de maîtriser l'équilibre entre un taux d'humidité résiduelle très faible et la conservation des facteurs de coagulation, qui peuvent s'avérer particulièrement sensible au procédé agressif de lyophilisation. Préférentiellement, l'étape c)de lyophilisation permet l'obtention d'un plasma lyophilisé présentant un taux d'humidité inférieur à 3%, préférentiellement inférieur à 2%. Typiquement, la lyophilisation de l'étape c) comprend plusieurs phases: congélation, dessiccation primaire ou sublimation, et dessiccation secondaire ou séchage final.

Par "lyophilisation" ou "cryodessication", on entend une opération de déshydratation à basse température qui consiste à éliminer par sublimation, la majeure partie de l'eau contenue dans un produit. Elle autorise une conservation à long terme grâce à l'abaissement de l'activité de l'eau du produit.

Un procédé de lyophilisation classique consiste en trois étapes :
1) Une étape de congélation : chaque formulation a une température critique pour la lyophilisation. Elles doivent être refroidies en dessous de ce point (température eutectique) pour une solidification optimale.
   Il existe deux types de congélation dépendant de la composition de la solution à congeler :
   - Une vitesse de congélation lente permet d'obtenir une structure de solution congelée présentant des cristaux de tailles importantes
   - Une vitesse de congélation rapide permet d'obtenir une structure de solution congelée avec de fins cristaux.
2) Une étape de dessiccation primaire dans laquelle la pression de la chambre de séchage (lyophilisation) est abaissée permettant la sublimation de la glace. Cette phase est effectuée à faible ou forte température selon la température de collapse ou de fusion du produit. La sublimation nécessite de l'énergie, la chaleur est transférée au produit à travers les étagères où reposent les flacons et par radiation. Cette étape permet la sublimation de l'eau dite « libre ». La structure de la solution congelée (donc la vitesse de congélation) influence la vitesse de sublimation, notamment en modifiant la pression de vapeur nécessaire au phénomène de sublimation.
3) Une étape de dessiccation secondaire (désorption) : la température des étagères est augmentée ou maintenue et la pression de la chambre est abaissée pour permettre la désorption de l'eau dite « liée » au produit. Ce qui a pour but d'obtenir une quantité finale d'humidité adaptée à la conservation du produit.

La lyophilisation est un procédé consommateur de temps et d'énergie qui peut prendre plusieurs jours ou même plusieurs semaines pour arriver à son terme (si le cycle de lyophilisation n'est pas optimisé). Le développement et l'optimisation des cycles de lyophilisation sont établis à partir des températures critiques (T'g Température de solidification, Tc Température de collapse, Tf température de fusion).

Dans un mode de réalisation particulier de l'invention, la lyophilisation comporte une étape préliminaire de prérefroidissement, avantageusement à -5°C.

Dans un mode de réalisation particulier de l'invention, la lyophilisation comporte une phase de congélation rapide entre -30°C et -60°C dans laquelle l'eau contenue dans le plasma est solidifiée. Typiquement, cette étape de congélation a une durée de quelques heures, avec une étape de diminution progressive de la température pour atteindre la température de solidification totale du plasma à lyophiliser.

En particulier, la phase de congélation rapide peut se dérouler à -50°C. L'étape de congélation s'effectue avec une rampe d'une durée comprise entre 15 minutes et 60 minutes, préférentiellement d'environ 30 minutes et un palier d'une durée comprise entre 100 et 600 minutes, préférentiellement d'environ 300 minutes.

Lorsque tout le plasma est congelé, intervient alors la phase de sublimation, également appelée dessiccation primaire, qui va entraîner le passage de l'eau de la forme solide à la forme vapeur, sans passage par une forme liquide. Cette étape se fait à basse pression et à une température inférieure à la température de fusion commençante, soit à une température comprise entre -20°C et 20°C. Typiquement, l'étape de dessiccation primaire dure quelques heures. La phase de dessiccation primaire est généralement prolongée, c'est-à-dire que la sublimation est continuée de manière à introduire une étape de sécurité dans le processus pour assurer une homogénéité du produit, notamment dans les cycles courts.

L'étape de dessiccation secondaire se fait sous une pression inférieure à 300 µBar (soit 30 Pa) et à une température comprise entre 10 et 15°C. Typiquement, le premier palier à 10°C a une rampe d'une durée comprise entre 20 et 120 minutes, préférentiellement d'environ 60 minutes et un palier d'une durée comprise entre 2000 et 4000 minutes, préférentiellement d'environ 3000 minutes. Le second palier à 15°C a une rampe d'une durée comprise entre 5 et 60 minutes, préférentiellement d'environ 10 minutes et un palier d'une durée comprise entre 800 et 2000 minutes, préférentiellement d'environ 1200 minutes.

Lorsque la phase de dessiccation primaire est terminée, le produit obtenu contient encore de l'eau résiduelle captive qui correspond aux molécules d'eau restant piégées en surface d'un produit soumis à une dessiccation primaire. La phase de dessiccation secondaire ou séchage final permet la désorption de ladite eau résiduelle pour obtenir une humidité du produit final minimale, à un taux inférieur ou égal à 3% en poids, en particulier inférieur ou égale à 2% en poids, en vue de la conservation du produit.

Cette étape de séchage final s'effectue à une température comprise entre 20 et 50°C sous une pression réduite.

En particulier, le séchage final s'effectue à une température comprise entre 30 et 35°C sous une pression réduite de 30 µBar (soit 3 Pa) environ. Typiquement, le premier palier à 35°C a une rampe d'une durée comprise entre 2000 et 15000 minutes, préférentiellement 6000 minutes et un palier d'une durée comprise entre 800 et 2000 minutes, préférentiellement 1200 minutes. Le second palier à 30°C a une rampe d'une durée comprise entre 2000 et 1000 minutes, préférentiellement 480 minutes et un palier d'une durée comprise entre 1200 et 2500 minutes, préférentiellement 1800 minutes.

Les phases de dessiccation primaire et secondaire ont des durées adaptées à la température critique du produit à lyophiliser (plasma).

Avantageusement, le protocole de lyophilisation utilisé permet l'obtention d'un plasma universel lyophilisé présentant un taux d'humidité inférieur à 3%, préférentiellement inférieur à 2%.

Par « atomisation » on entend une méthode de déshydratation d'un liquide (lait, sérum, plasma, etc.) sous forme de poudre par passage dans un flux d'air chaud.

Le procédé de l'invention, indépendamment de la présence ou non d'une l'étape de lyophilisation ou de cryodessiccation ou d'atomisation peut en outre comprendre une étape de sécurisation biologique en amont de l'étape a) dudit procédé. Les plasmas unitaires non universels constituant le mélange de plasmas de l'étape a) du procédé de l'invention sont sécurisés biologiquement, par exemple viro-inactivés par traitement physico-chimique. Par "sécurisation biologique", on entend la suppression de l'effet pathogène d'agents viraux susceptibles d'être présents dans le plasma. La "sécurisation biologique" peut notamment recouvrir "l'inactivation virale" ou "la viro-inactivation". Cette sécurisation biologique détruit la majorité des agents pathogènes tels que des bactéries, virus enveloppés ou non-enveloppés, ou autres agents pathogènes non conventionnels (prions) ou empêche leur réplication.

Dans le contexte de cette invention, la sécurisation biologique se fait préférentiellement par traitement physico-chimique des plasmas unitaires non universels, préalablement à leur mélange. Alternativement, cette sécurisation biologique peut se faire sur le mélange de plasmas obtenu à l'étape a). La sécurisation biologique par traitement physico-chimique peut être un traitement à l'aide d'un agent photochimique (UV, IR...) ou par solvant-détergent.

Dans un mode de réalisation particulier de l'invention, la sécurisation biologique est effectuée par addition d'Amotosalem (Intercept®) et irradiation UV 5-10min à 380-400nm.

Préférentiellement, cette sécurisation biologique s'effectue par traitement solvant/détergent. De préférence le solvant/détergent utilisé est un solvant/détergent dont l'élimination du plasma d'intérêt est facilitée du fait de ses propriétés, par exemple un solvant/détergent dialysable ou un solvant/détergent filtrable. En effet, l'utilisation de solvant/détergent dialysable et/ou filtrable permet de faciliter l'élimination de ces produits, contrairement aux solvants/détergents non dialysables et/ou non filtrables (comme le TNBP/Triton X-100®) qui nécessitent des étapes dédiées d'élimination par exemple par extraction à l'huile végétale ou adsorption sur chromatographie hydrophobe. On peut citer comme solvant/détergent dialysable et/ou filtrable par exemple l'HECAMEG® (6-O-(N-Heptylcarbamoyl)-methyl-α-D-glucopyranoside).

Avantageusement, l'étape de sécurisation biologique est couplée si nécessaire à une étape d'élimination du traitement, par exemple par dialyse, par diafiltration, par filtration sur filtre d'affinité (Plasmaflex PLAS4®, Macopharma) ou en utilisant un dispositif dédié (Solvent Detergent Removal® de Pall Biosepra) ou une résine adsorbante (résine SDR hyperD® commercialisée par la société Pall Life Sciences). Dans un mode de réalisation particulier, l'étape d'élimination du traitement est réalisée à une température comprise entre 0 et 25°C, avantageusement entre 5 et 20°C, encore plus avantageusement entre 10 et 15°C. Des températures inférieures à 25°C, notamment inférieurs à 20°C, permettent avantageusement de limiter partiellement ou totalement l'activation des facteurs de coagulation.

Dans un autre mode de réalisation particulier de l'invention, l'étape de sécurisation biologique est effectuée par tout moyen approprié connu de l'homme du métier, notamment par des méthodes d'inactivation ou d'élimination virale. Par méthodes d'inactivation virale on entend notamment le traitement par la chaleur (pasteurisation et/ou chauffage à sec), et/ou par irradiation (UVC, et/ou Gamma).

Les méthodes d'élimination virale incluent notamment la nanofiltration qui peut également être utilisée pour éliminer un agent infectieux, notamment les virus et les

ATNC. La nanofiltration se réfère généralement à la filtration du plasma à travers un filtre avec une taille de pores inférieure à 80 nm. Les filtres disponibles sont par exemple les filtres BioEx®, Planova® 75 nm, Planova® 35 nm, Planova® 20 nm ou Planova® 15 nm (Asahi corporation), Ultipor DV 50® ou DV 20® (Pall corporation), Virosart CPV® (Sartorius), Viresolve NFR® ou NFP® (Millipore). La nanofiltration peut être avantageusement effectuée sur un filtre unique ou sur plusieurs filtres en série de porosité identique ou décroissante, par exemple avec une séquence 35nm-35nm, 35nm-20nm, 20nm-20nm ou 20nm-15nm.

L'élimination des agents infectieux peut également être réalisée au moyen d'une filtration en profondeur sur des filtres. Les filtres disponibles sont par exemple des filtres composés de cellulose régénérée, dans lesquels des adjuvants de filtration peuvent avoir été additionnés (tels que la cellite, la perlite ou des terres de Kieselguhr) commercialisés par Cuno (filtres Zeta+ VR series®), Pall-Seitz® (P-series Depth Filter®) ou Sartorius (Virosart CPV®, Sartoclear P depth filters®).

Par "agent pathogène ", on entend un contaminant viral, bactérien ou de type pathogène non conventionnel (prion). La présence de tels contaminants est inacceptable pour l'utilisation d'un plasma en thérapeutique.

Par "plasma sécurisé biologiquement" ou "plasma viro-inactivé" on entend un plasma ayant subi une étape de sécurisation biologique, par exemple par inactivation, c'est-à-dire la destruction réelle ou l'inhibition de la réplication d'agents pathogènes tels que des contaminants viraux, bactériens ou pathogènes non conventionnels. Dans un mode de réalisation particulier, le mélange de plasmas de l'étape a) du procédé de l'invention sont inactivés par solvant/détergent. Une telle technique d'inactivation du plasma est particulièrement avantageuse puisqu'elle permet la sécurisation biologique du plasma par simple ajout de solvant/détergent dans des conditions permettant la conservation de l'intégrité des autres constituants du plasma. Les conditions opératoires permettant l'élimination d'agents pathogènes à l'aide de solvant/détergent tout en préservant les autres constituants du plasma sont connues de l'homme du métier. Selon l'invention, cette technique d'élimination d'agents pathogènes est préférentiellement appliquée à chaque plasma unitaire recueilli par aphérèse. Dans un autre mode de réalisation, la technique d'élimination d'agents pathogènes est avantageusement appliquée au pool de plasma.

Dans un mode de réalisation particulier de l'invention, une étape supplémentaire peut être ajoutée pour ajuster les propriétés physico-chimiques et restaurer des propriétés physico-chimiques similaires ou proches de celles d'un plasma non traité. En particulier, l'équilibre ionique, le pH, l'osmolarité, doivent être maintenus à des niveaux physiologiques compatibles avec l'administration aux patients. Hors selon les méthodes utilisées dans le procédé selon l'invention, certains paramètres, comme le ionogramme, peuvent être légèrement altérés, notamment par exemple lors d'étapes de diafiltration. Une étape permettant de restaurer les propriétés physico-chimiques du plasma normal, par exemple l'ionogramme, sont donc particulièrement avantageuses.

Le procédé de l'invention peut également comprendre une étape de filtration stérilisante ou filtration clarifiante sur un filtre de 0,45 µm et/ou 0,2 µm, par exemple sur filtre polypropylène ou média équivalent tel que le filtre ProFile (Pall).

Le procédé de l'invention peut en outre comprendre une étape d'ultrafiltration en amont de l'étape a) dudit procédé visant à éliminer les bactéries et le solvant-détergent éventuellement utilisé pour inactiver le plasma. Les plasmas unitaires constituant le mélange de plasmas de l'étape a) du procédé de l'invention sont ultrafiltrés afin d'éliminer les bactéries potentiellement présentes dans le plasma. L'ultrafiltration peut avantageusement comprendre une étape de dialyse pour éliminer le solvant-détergent utilisé pour l'inactivation du plasma.

Dans le contexte de cette invention, l'élimination des bactéries se fait par ultrafiltration des plasmas unitaires, préalablement à leur mélange. Alternativement, cette élimination bactérienne peut se faire sur le mélange de plasmas obtenu à l'étape a).

Selon l'invention, cette technique d'élimination des bactéries par ultrafiltration est préférentiellement appliquée à chaque plasma unitaire recueilli par aphérèse. Dans un autre mode de réalisation de l'invention, l'élimination des bactéries par ultrafiltration est appliquée sur le pool de plasma.

Dans un mode de réalisation particulier de l'invention, l'étape d'ultrafiltration est réalisée de manière à limiter totalement ou partiellement l'activation des facteurs de coagulation.

La présente invention concerne également un plasma universel obtenu selon le procédé décrit ci-dessus.

L'invention concerne également un plasma universel lyophilisé et compatible avec tous les groupes sanguins. Préférentiellement, le plasma universel lyophilisé de l'invention est caractérisé en ce qu'il comprend un mélange de plasmas recueillis à partir d'individus donneurs appartenant aux groupes sanguins A, B, AB et/ou O. Ce plasma lyophilisé présente un taux d'humidité inférieur à 3%, préférentiellement inférieur à 2%. Il est en outre caractérisé en ce qu'il peut être stocké à température ambiante ou dans une enceinte réfrigérée à une température comprise entre +2°C et +30°C et pour une durée supérieure à un an, préférentiellement d'au moins 2 ans.

Préférentiellement, le plasma universel lyophilisé de l'invention est caractérisé en ce qu'il comprend une teneur en anticorps anti-A et/ou anti-B telle que le résultat du test de Coombs est négatif à la dilution 1/64. De préférence, la teneur en anticorps anti-A du plasma universel lyophilisé de l'invention est telle que le résultat du test de Coombs est négatif à la dilution 1/32, de préférence négatif à la dilution 1/16, encore plus préférentiellement négatif à la dilution 1/8 ou inférieure. De préférence, la teneur en anticorps anti-B du plasma universel lyophilisé de l'invention est telle que le résultat du test de Coombs est négatif à la dilution 1/32, de préférence négatif à la dilution 1/16, encore plus préférentiellement négatif à la dilution 1/8 ou inférieure Préférentiellement, ce plasma sanguin lyophilisé est stérile.

Avantageusement, le plasma sanguin lyophilisé est appauvri en endotoxines.

L'invention concerne également un procédé de préparation de plasma reconstitué comprenant l'étape de reconstitution du plasma lyophilisé, sécurisé biologiquement, et compatible avec tous les groupes sanguins dans un solvant de reprise. La reconstitution du plasma permet ainsi l'obtention d'une préparation injectable, susceptible d'être administrée à n'importe quel receveur dans des conditions d'urgence.

Typiquement, la reconstitution du plasma s'effectue dans un volume de solvant de reprise compris entre 10 et 500 mL, préférentiellement de 100-300 mL. Typiquement, cette reconstitution s'effectue avec un volume permettant d'obtenir un plasma iso-osmotique.

Préférentiellement, ce solvant de reprise est l'eau et plus préférentiellement de l'eau pour préparation injectable. Préférentiellement, la reconstitution du plasma lyophilisé pour l'obtention d'une préparation injectable s'effectue en une durée inférieure à 6 minutes, préférentiellement inférieure à 3 minutes.

Aussi, l'utilisation du plasma selon l'invention est très avantageuse et s'affranchit du temps nécessaire à la décongélation lors de l'utilisation de plasma frais congelé.

L'invention concerne également un plasma reconstitué, sécurisé biologiquement, et compatible avec tous les groupes sanguins et directement injectable. Un tel plasma reconstitué est susceptible d'être administré à n'importe quel individu, indépendamment de son groupe sanguin. Il est donc hautement adapté pour une utilisation dans des conditions d'urgence, notamment pour le traitement des urgences hémorragiques avec coagulopathie, notamment en situation isolée avec des conditions logistiques ne permettant pas de maîtriser une chaîne du froid négative.

Le plasma reconstitué selon l'invention présente en outre pour avantage la destruction de la plupart des agents pathogènes ce qui réduit considérablement la diffusion potentielle de pathogènes aux individus receveurs. Ce plasma reconstitué répond à toutes les exigences réglementaires auxquelles sont soumis les plasmas utilisés en thérapeutique.

Le plasma reconstitué de l'invention est caractérisé en ce que la concentration en facteur VIII est supérieure à 0,5 UI/mL, préférentiellement supérieure à 0,7 UI/mL.

Le plasma reconstitué de l'invention est caractérisé en ce que la concentration en facteur V est supérieure à 0,15 UI/mL, et préférentiellement comprise entre 0,7 et 1,2 UI/mL. Les unités internationales (UI) pour les facteurs de coagulation expriment l'activité plasmatique des protéines auxquelles cette expression est appliquée. Une unité internationale (UI) de ces protéines plasmatiques correspond à la quantité de ce facteur contenue dans un mL de plasma humain normal.

Le plasma reconstitué de l'invention est caractérisé en ce que la concentration en fibrinogène est supérieure à 1 g/L et plus préférentiellement comprise entre 2 et 4 g/L.

Le plasma reconstitué de l'invention est caractérisé en ce qu'il est stérile et apyrogène.

Bien entendu, la présente invention n'est pas limitée aux modes de réalisation décrits et représentés, mais elle est susceptible de nombreuses variantes accessibles à l'homme de l'art.

### Figures

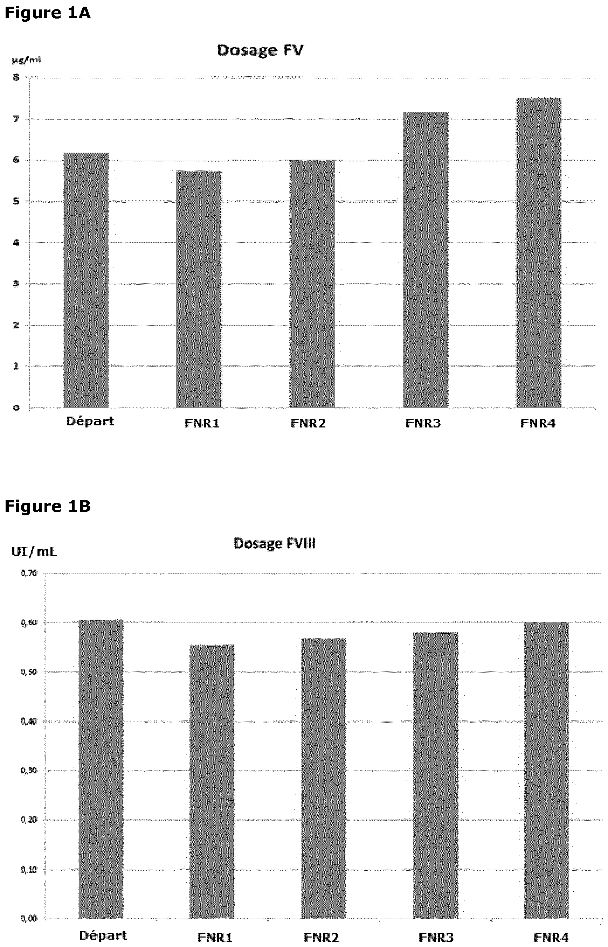

Figures 1A et 1B: ces figures illustrent les résultats respectifs de dosage du facteur V (figure 1A) et du facteur VIII (figure 1B) présents dans le plasma universel obtenu à partir d'individus donneurs humains appartenant aux groupes sanguins A, B, AB et O après la chromatographie d'immunoaffinité, dans le plasma de départ et les fractions non retenues (FRN 1-4).

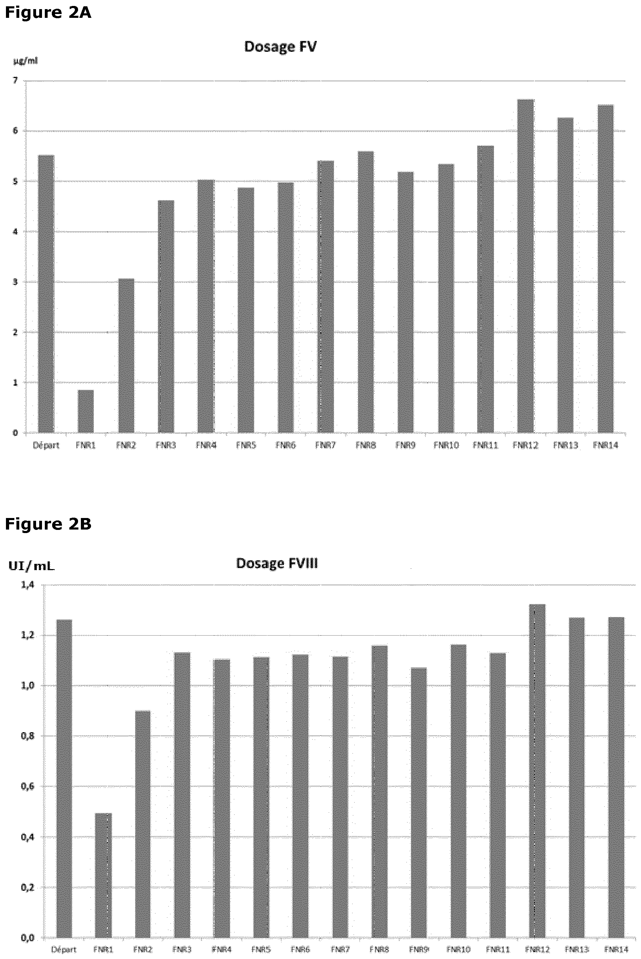

Figure 2A et 2B: ces figures illustrent les résultats respectifs de dosage du facteur V (figure 2A) et du facteur VIII (figure 2B) présents dans le plasma universel obtenu à partir d'individus donneurs humains appartenant au groupe sanguin O après la chromatographie d'immunoaffinité, dans le plasma de départ et les fractions non retenues (FRN 1-14).

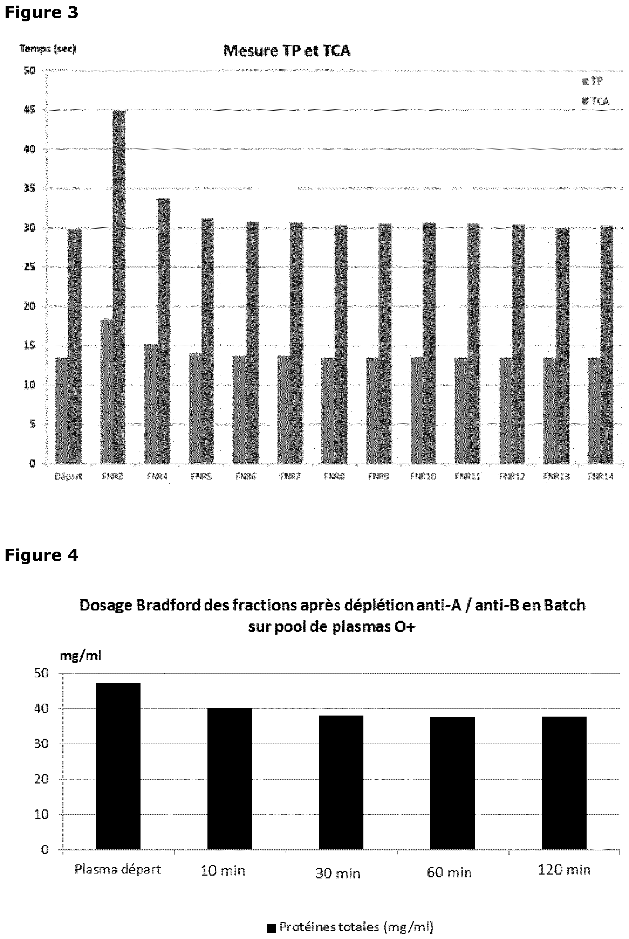

Figure 3 montre les résultats de mesure de temps de Quick (TP) et de mesure du temps de céphaline activé (TCA) du plasma universel obtenu à partir d'individus donneurs humains appartenant au groupe sanguin O après la chromatographie d'immunoaffinité, dans le plasma de départ et les fractions non retenues (FRN1-14).
Figure 4 montre le résultat de dosage de protéines totales présentes dans le plasma universel obtenu à partir d'individus donneurs humains appartenant au groupe sanguin O après la déplétion en batch, dans le plasma de départ et les prélèvements respectivement après 10 min, 30 min, 1h et 2h d'incubation.

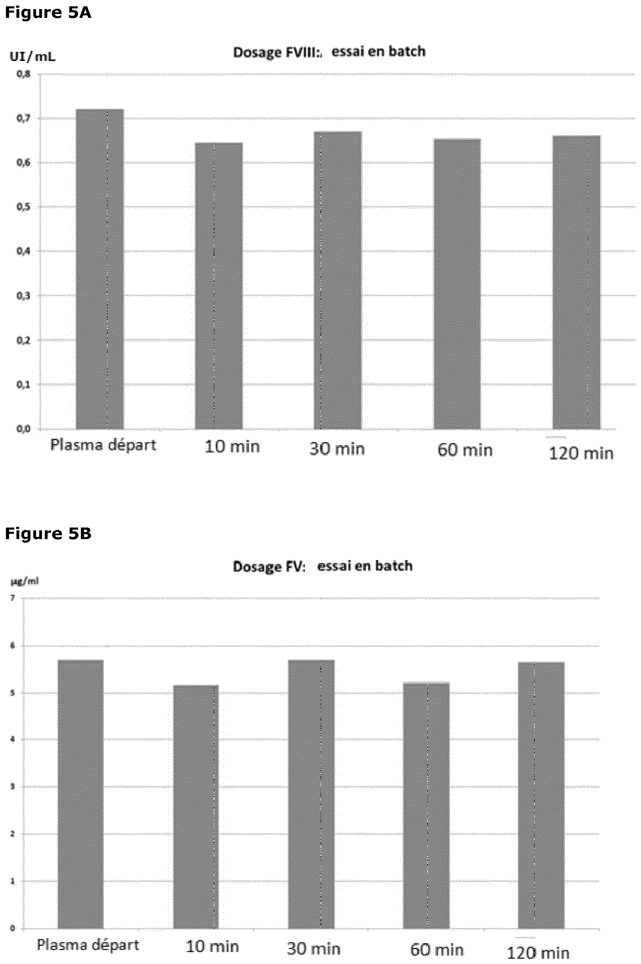

Figure 5A et 5B : ces figures illustrent les résultats respectifs de dosage du facteur VIII (figure 5A) et du facteur V (figure 5B) présents dans le plasma universel obtenu à partir d'individus donneurs humains appartenant au groupe sanguin O après la déplétion en batch, dans le plasma de départ et les prélèvements respectivement après 10 min, 30 min, 1h et 2h d'incubation.

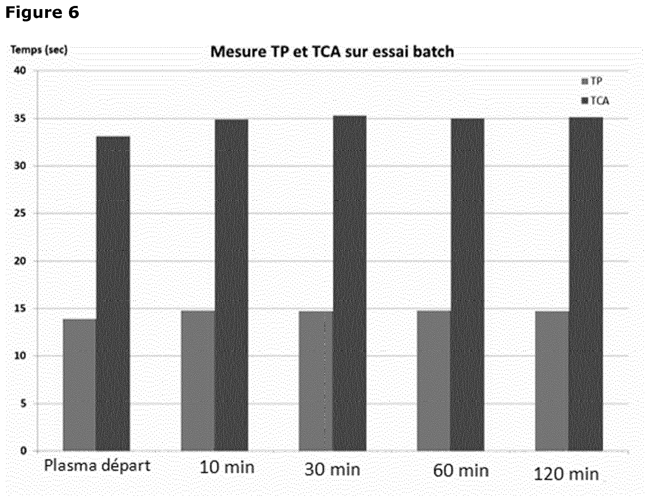

Figure 6 montre les résultats de mesure de temps de quick et de mesure du temps de céphaline activé du plasma universel obtenu à partir d'individus donneurs humains appartenant au groupe sanguin O après la déplétion en batch, dans le plasma de départ et les prélèvements respectivement après 10 min, 30 min, 1h et 2h d'incubation.

### EXEMPLES

### Exemple 1: Préparation de plasma universel obtenu à partir d'individus donneurs humains appartenant aux groupes sanguins A, B, AB et O

### A/ Collecte des plasmas unitaires non universels

On recueille par aphérèse environ 200 mL de plasma d'individus donneurs humains appartenant aux groupes sanguins A, B, AB et O.

Lors de la collecte du plasma, on procède à la déleucocytation desdits plasmas unitaires non universels par centrifugation à deux reprises à 1500g pendant 10 min.

Les plasmas unitaires non universels sont ensuite soumis à une étape de sécurisation biologique par traitement à l'Amotosalem (Intercept®) : 15mL d'amotosalem à 150µM sont ajoutés à chaque plasma unitaire non universel qui sont ensuite soumis à 5-10min d'irradiation par UVA à une longueur d'onde de 380-400nm. L'Amotosalem résiduel ainsi que les photoproduits sont ensuite éliminés par filtration sur filtre adsorbant pour atteindre un taux résiduel d'Amotosalem < 2µM.

On procède ensuite à la surgélation desdits plasmas unitaires non universels atténués dans les 8 heures suivant la collecte des plasmas. Cette étape permet alors la conservation desdits plasmas à une température de -25°C. 15 minutes avant de procéder au mélange des plasmas, on place les plasmas à décongeler dans un bain marie à 37°C.

### B/ Mélange des plasmas unitaires non universels

Le mélange de plasmas est préparé en mélangeant les plasmas unitaires non universels obtenus à partir d'individus donneurs humains appartenant aux groupes sanguins A, B, AB et O. Par exemple, pour le mélange, on utilise :
- 6 poches de plasmas (1327 mL de plasma) obtenus à partir de 3 individus donneurs différents appartenant au groupe sanguin A,
- 6 poches de plasmas (1327 mL de plasma) obtenus à partir de 3 individus donneurs différents appartenant au groupe sanguin B, et
- 6 poches de plasmas (1327 mL de plasma) obtenus à partir de 3 individus donneurs différents appartenant au groupe sanguin AB, et
- 6 poches de plasmas (1327 mL de plasma) obtenus à partir de 3 individus donneurs différents appartenant au groupe sanguin O.

On obtient ainsi un mélange comprenant 5308 mL de plasmas non universel.

### C/ Etape d'élimination des anticorps anti-A et anti-B présents dans le plasma.

Le mélange de plasmas non universel obtenu à l'étape précédente est soumis à une échelle industrielle, à une étape de chromatographie d'affinité anti-A/ anti-B réalisée sur une colonne comprenant un mélange 50/50 (v/v) de billes de cellulose réticulée sur lesquelles sont greffés des trisaccharides correspondant à un épitope du groupe sanguin A (N-acétylgalactosamine (GalNAc)-Galactose(Gal)-Fucose), désigné gel Iso A HyperCel®, et de billes de cellulose réticulée sur lesquelles sont greffés des trisaccharides correspondant à un épitope du groupe sanguin B (Galactose-Galactose-Fucose), désigné gel Iso B HyperCel®.

Les trisaccharides sont greffés aux billes par un espaceur de formule :

-NH-C₅H₁₀-CO-NH-C₃H₆-

La matrice utilisée présente la formule suivante :
(particule de polymère)- NH-C₅H₁₀-CO-NH-C₃H₆-(N-acétylGalα₁₋₃(Fucα₁₋₂)Gal)
**et** (particule de polymère)- NH-C₅H₁₀-CO-NH-C₃H₆- (Galα1-3(Fuc α₁₋₂)Gal)

La densité de trisaccharides greffés est de 0,3mg par ml de gel de matrice.

Le mélange de plasma a été passé sur une colonne de 5,5 ml (comprenant 2,75 ml de gel Iso A HyperCel® + 2,75 ml de gel Iso B HyperCel®)

Format de la colonne : D = 1 cm x 7 cm (volume de la colonne (VC) = 5,5 ml).
La colonne est équilibrée en tampon phosphate salin (PBS).
Temps de contact : 2,5 min
Charge : 165mL de mélange de plasma à un débit de 2mL/min.
L'effluent (4 fractions non retenues) est récupéré en fractions d'environ 45mL. La collecte est arrêtée à la fin de l'injection pour éviter la dilution du plasma.

### Tests de dosage :

L'activité résiduelle anti-A et anti-B, correspondant au pourcentage d'isoagglutinines anti-A et anti-B présentes dans la préparation de plasma finale par rapport à leur concentration avant l'étape de chromatographie d'affinité, a été mesurée par cytométrie en flux (technique sensible et précise), selon la technique décrite ci-dessous.

Les puits d'une plaque fond V sont saturés par 125 µl de PBS additionnés de 2% de sérum de veau foetal (SVF) pendant 1 heure à 37 °C. 100 µl de suspension de globules rouges de groupe AB sont ensuite ajoutés (PBS + 2% SVF) à raison de 0,05 % par puits. Les plaques sont centrifugées 1 min. à 100g et le surnageant est éliminé.
100 µl de plasma natif ou de fraction non retenue (FNR) +/- dilué en PBS + 2% SVF sont ajoutés avant incubation 60 min. à 37°C. 6 lavages sont effectués par addition de 150 µl en PBS + 2% SVF.
On ajoute ensuite 100 µl d'anticorps secondaire Anti IgG-PE en PBS + 2% SVF et on laisse incuber 20 min. à +4 °C.
Après 2 lavages en PBS + 2% SVF, l'échantillon est lu au cytométrie en flux.

L'intensité moyenne de fluorescence (IMF) du contrôle positif a été rapportée en fonction de la concentration en plasma (courbe standard) pour des concentrations allant de 0,23 g/L à 30 g/L. Les résultats sont exprimés comme le rapport entre la pente de l'échantillon et la pente du standard positif. L'équation de la courbe standard est y = ax + b; où "a" est la valeur de la pente de la courbe standard et "b", le point zéro correspondant au bruit de fond de l'essai. Comme l'équation de l'échantillon est y' = a'x + b, et en utilisant les valeurs connues de IMF de l'échantillon (y') et la concentration en plasma (x'), le rapport des pentes a été calculé comme étant [(IMF-b) / [IgG concentration]] / a.

### Résultats :

L'activité résiduelle anti-A et anti-B obtenue est présentée dans le tableau 1 ci-dessous

**Tableau 1**

| Fraction | % d'agglutination (IgG) |
|---|---|
| Plasma de départ | 100% |
| FNR1 | 6% |
| FNR2 | 9% |
| FNR3 | 16% |
| FNR4 | 21% |

Les résultats montrent une réduction importante de l'activité résiduelle anti-A et anti-B (de type IgG), qui est comprise entre 6 et 21% après immunoaffinité.

L'activité résiduelle anti-A et anti-B a également été mesurée par test de Coombs selon la méthode suivante :
100 µl de suspension de globules rouges AB à 1 % en eau physiologique sont déposés par puits sur microplaque puis centrifugés 1 min. à 100g. Le surnageant est éliminé puis on ajoute 100 µl de plasma ou de fraction non retenue (FNR) +/- dilué en eau physiologique. Après incubation 30 min. à 37°C et centrifugation 1 min. à 100g, les agglutinats sont lus par agitation douce.

Les résultats sont illustrés dans le tableau 2 ci-après.

**Tableau 2**

| | Agglutinats | | | | | |
|---|---|---|---|---|---|---|
| Dilutions | Plasma de Départ | FNR1 | FNR2 | FNR3 | FNR4 | Sérum AB |
| Dilution au 1/1 | + | + | + | + | + | - |
| Dilution au 1/2 | + | + | + | + | + | - |
| Dilution au 1/4 | + | + | + | + | + | - |
| Dilution au 1/8 | + | - | + | + | + | - |
| Dilution au 1/16 | + | - | - | + | + | - |
| Dilution au 1/32 | + | - | - | - | - | - |
| Dilution au 1/64 | - | - | - | - | - | - |
| | | | | | | |
| Dilution au 1/128 | - | - | - | - | - | - |
| Dilution au 1/256 | - | - | - | - | - | - |
| Dilution au 1/512 | - | - | - | - | - | - |

Les résultats du tableau 2 montrent que le taux exprimé en test de Coombs direct dans les fractions 1 à 4 est compris entre ¼ et 1/16.
Le produit ainsi obtenu est alors conforme (à la dilution 1/64) à un résultat négatif au test de Coombs direct.
On obtient ainsi un plasma universel appauvri en anticorps anti-A et anti-B.

### Teneur en facteurs de la coagulation présents dans le plasma universel

L'activité des facteurs de coagulation contenus dans le plasma universel résultant de l'étape de chromatographie d'immunoaffinité a été testée. Les modes opératoires et les résultats sont présentés ci-après.

### Dosage du facteur V

Le facteur V (FV) a été dosé par le test Zymutest Factor V de Hyphen Biomed, France (ref : RK009A) comme recommandé par le fabricant. L'essai est un ELISA sandwich qui utilise un anticorps anti-FV fixé sur des micropuits. Le plasma citraté est dilué dans le tampon fourni (1/100 et1/200) et incubé 2h à 37°C. La plaque est lavée 5 fois et incubée avec un anticorps secondaire anti-FV couplé à la peroxydase de raifort (horse radish peroxydase) pour 2h à 37°C. La plaque est lavée 5 fois et l'anticorps lié est révélé pendant 5 min avec une solution de 3,3',5,5'-tétraméthylbenzidine (TMB). La réaction est arrêtée en ajoutant H₂SO₄ 0,45 M. La densité optique est lue à 450nm après 10 min de stabilisation. Une courbe étalon est établie et deux plasmas contrôles avec des concentrations intermédiaires de FV sont réalisés à chaque essai.

### Dosage du facteur VIII

Le facteur VIII (FVIII) a été dosé par le test Asserachrom® VIII:Ag obtenu de Stago, France (ref: 00280) comme recommandé par le fabricant. L'essai est un ELISA sandwich qui utilise un anticorps anti-FVIII fixé sur des micropuits. Le plasma citraté est dilué dans le tampon fourni (1/10 et1/20) et incubé 2h à température ambiante. La plaque est lavée 5 fois et incubée avec un anticorps secondaire anti-FVIII couplé à la peroxydase de raifort (horse radish peroxydase) pour 2h à température ambiante. La plaque est lavée 5 fois et l'anticorps lié est révélé pendant 5 min avec une solution de TMB. La réaction est arrêtée en ajoutant H₂SO₄ 0,45 M. La densité optique est lue à 450 nm après 10 min de stabilisation. Une courbe étalon est établie et un plasma contrôle avec une concentration connue de FVIII est réalisé à chaque essai.

### Résultats :

Les résultats respectifs de dosage du facteur V et du facteur VIII sont illustrés dans les figures 1A et 1B.

Le passage sur colonne de chromatographie d'immunoaffinité n'affecte pas les taux en facteur V et en facteur VIII du plasma. Le plasma universel obtenu présente un taux de facteur V supérieur à 5µg/mL et un taux de facteur VIII supérieur à 0.5 UI/mL.

### Exemple 2: Préparation du plasma universel à partir d'individus donneurs humains appartenant au groupe sanguin O à l'aide de chromatographie d'immunoaffinité

### A/ Collecte des plasmas unitaires non universels

On recueille par aphérèse environ 500 mL de plasma d'individus donneurs humains appartenant au groupe sanguin O.

Lors de la collecte du plasma, on procède à la déleucocytation desdits plasmas unitaires non universels par centrifugation à deux reprises 1500g pendant 10 min.

Les plasmas unitaires non universels sont ensuite soumis à une étape de sécurisation biologique par traitement à l'Amotosalem (Intercept®) : 15mL d'amotosalem à 150µM sont ajoutés à chaque plasma unitaire non universel qui est ensuite soumis à 5-10min d'irradiation par UVA à une longueur d'onde de 380-400nm. L'Amotosalem résiduel ainsi que les photoproduits sont ensuite éliminés par filtration sur filtre adsorbant pour atteindre un taux résiduel d'Amotosalem inférieur à 2µM.

On procède ensuite à la surgélation desdits plasmas unitaires non universels atténués dans les 8 heures suivant la collecte des plasmas. Cette étape permet alors la conservation desdits plasmas à une température de -25°C.

15 minutes avant de procéder au mélange des plasmas, on place les plasmas à décongeler dans un bain marie à 37°C.

### B/ Mélange des plasmas unitaires non universels

Le mélange de plasmas est préparé en mélangeant trois plasmas unitaires non universels de 75mL obtenus à partir de 3 individus donneurs humains appartenant aux groupes sanguins O.

On obtient ainsi un mélange comprenant 225mL de plasma non universel.

### C/ Etape d'élimination des anticorps anti-A et anti-B présents dans le plasma.

Le mélange de plasmas non universel obtenu à l'étape précédente est soumis à une échelle industrielle, à une étape de chromatographie d'affinité anti-A/ anti-B réalisée sur une colonne comprenant un mélange 50/50 (v/v) de billes de cellulose réticulée sur lesquelles sont greffés des trisaccharides correspondant à un épitope du groupe sanguin A (N-acétylgalactosamine (GalNAc)-Galactose(Gal)-Fucose), désigné gel Iso A HyperCel®, et de billes de cellulose réticulée sur lesquelles sont greffés des trisaccharides correspondant à un épitope du groupe sanguin B (Galactose-Galactose-Fucose), désigné gel Iso B HyperCel®.

Les trisaccharides sont greffés aux billes par un espaceur de formule :

-NH-C₅H₁₀-CO-NH-C₃H₆-.

La matrice utilisée présente la formule suivante :
(particule de polymère)- NH-C₅H₁₀-CO-NH-C₃H₆-(N-acétylGalα₁₋₃(Fucα₁₋₂)Gal)
et (particule de polymère)- NH-C₅H₁₀-CO-NH-C₃H₆- (Galα1-3(Fuc α₁₋₂)Gal).

La densité de trisaccharides greffés est de 0,3mg par ml de gel de matrice.
Le mélange de plasma a été passé sur une colonne de 5,5 ml (comprenant 2,75 ml de gel Iso A HyperCel® + 2,75 ml de gel Iso B HyperCel®)
Format de la colonne : D = 1 cm x 7 cm (volume de la colonne (VC) = 5,5 ml).
La colonne est équilibrée en tampon PBS.
Temps de contact : 5 min
Charge : 20mL de mélange de plasma à un débit de 1mL/min. L'effluent (14 fractions non retenues) est récupéré en fractions de 1mL. La collecte est arrêtée à la fin de l'injection pour éviter la dilution du plasma.

### Tests de dosage :

L'activité résiduelle anti-A et anti-B, correspondant au pourcentage d'isoagglutinines anti-A et anti-B présentes dans la préparation de plasma finale par rapport à leur concentration avant l'étape de chromatographie d'affinité, a été mesurée par cytométrie en flux (technique sensible et précise), selon la technique décrite ci-dessous.

Les puits d'une plaque fond V sont saturés par 125 µl de PBS additionnés de 2% de sérum de veau foetal (SVF) pendant 1 heure à 37 °C. 100 µl de suspension de globules rouges AB sont ensuite ajoutés (PBS + 2% SVF) à raison de 0,05 % par puits. Les plaques sont centrifugées 1 min. à 100g et le surnageant est éliminé.

100 µl de plasma natif ou de fraction non retenue (FNR) +/- dilué en PBS + 2% SVF sont ajoutés avant incubation 60 min. à 37°C.
6 lavages sont effectués par addition de 150 µl en PBS + 2% SVF.
On ajoute ensuite 100 µl d'anticorps secondaire Anti IgM-PE ou Anti IgG-PE en PBS + 2% SVF et on laisse incuber 20 min. à +4 °C.
Après 2 lavages en PBS + 2% SVF, l'échantillon est lu au cytomètre.

L'intensité moyenne de fluorescence (IMF) du contrôle positif a été rapportée en fonction de la concentration en plasma (courbe standard) pour des concentrations allant de 0,23 g/L à 30 g/L. Les résultats sont exprimés comme le rapport entre la pente de l'échantillon et la pente du standard positif. L'équation de la courbe standard est y = ax + b; où "a" est la valeur de la pente de la courbe standard et "b", le point zéro correspondant au bruit de fond de l'essai. Comme l'équation de l'échantillon est y' = a'x + b, et en utilisant les valeurs connues de IMF de l'échantillon (y') et la concentration en plasma (x'), le rapport des pentes a été calculé comme étant [(IMF-b) / [IgG concentration]] / a.

### Résultats :

L'activité résiduelle anti-A et anti-B obtenue est présentée dans le tableau 3 :

**Tableau 3**

| Fraction | % d'agglutination (IgM) | % d'agglutination (IgG) |
|---|---|---|
| Plasma de départ | 100 | 100 |
| FNR1 | 9 | 7 |
| FNR2 | 11 | 9 |
| FNR3 | 12 | 9 |

Les résultats montrent une réduction importante des activités résiduelles anti-A et anti-B, qui sont comprises entre 9 et 12% (IgM) et entre 7 et 9% (IgG) après immunoaffinité. La chromatographie d'immunoaffinité permet donc d'éliminer au moins 80% des anti-A et des anti-B de types IgG et IgM d'un plasma, même lorsque le taux initial dans le plasma de départ est très faible.

L'activité résiduelle anti-A et anti-B a également été mesurée par test de Coombs selon la méthode suivante :
100 µl de suspension de globules rouges AB à 1 % en eau physiologique sont déposés par puits sur microplaque puis centrifugés 1 min. à 100g. Le surnageant est éliminé puis on ajoute 100 µl de plasma ou de fraction non retenue (FNR) +/- dilué en eau physiologique. Après incubation 30 min. à 37°C et centrifugation 1 min. à 100g, les agglutinats sont lus par agitation douce.

Les résultats sont illustrés dans le tableau 4 ci-après :

**Tableau 4**

| Dilutions | Agglutinats | | | | | | | | |
|---|---|---|---|---|---|---|---|---|---|
| | Plasma N°2 | Tube 3 | Tube 4 | Tube 5 | Tube 7 | Tube 10 | Tube 13 | Sérum AB | Plasma AB |
| 1/1 | + | - | + /- | + /- | + /- | + /- | + /- | - | - |
| 1/2 | + | - | - | - | - | - | - | - | - |
| 1/4 | +/- | - | - | - | - | - | - | - | - |
| 1/8 | - | - | - | - | - | - | - | - | - |
| 1/16 | - | - | - | - | - | - | - | - | - |
| 1/32 | - | - | - | - | - | - | - | - | - |
| 1/64 | - | - | - | - | - | - | - | - | - |
| 1/128 | - | - | - | - | - | - | - | - | - |

Les résultats montrent que même avec un taux initial très faible mesuré en test de Coombs (inférieur à 1/4 dans le plasma initial), la chromatographie d'immunoaffinité permet de réduire le taux d'anti-A et d'anti-B dans le plasma à un taux inférieur ou égal à 1/1 mesuré en test de Coombs.

Le produit ainsi obtenu est alors conforme (à la dilution 1/64) à un résultat négatif au test de Coombs direct.

On obtient ainsi un plasma universel appauvri en anticorps anti-A et anti-B.

### Teneur en facteurs de la coagulation présents dans le plasma universel

L'activité des facteurs de coagulation contenus dans le plasma universel résultant de l'étape de chromatographie d'immunoaffinité a été testée. Les résultats sont présentés ci-après.

### Dosage du facteur V

Le facteur V(FV) a été dosé selon le protocole de dosage décrit dans l'exemple 1.

### Dosage du facteur VIII

Le facteur VIII (FVIII) a été dosé selon le protocole de dosage décrit dans l'exemple 1.

### Résultats :

Les résultats respectifs de dosage du facteur V et du facteur VIII sont illustrés dans les figures 2A et 2B.

Le passage sur colonne de chromatographie d'immunoaffinité n'affecte pas les taux en facteur V et en facteur VIII du plasma. On note uniquement une légère baisse du taux de FVIII et de FV sur les premières fractions (FNR1-2) qui sont diluées par le tampon contenu dans le volume mort de la colonne. Les autres fractions ne sont pas affectées par ce phénomène de dilution.

Le plasma universel obtenu présente un taux de facteur V supérieur à 4µg/mL et un taux de facteur VIII supérieur à 0,5 UI/mL.

Ceci est par ailleurs confirmé par les mesures de temps de Quick et de temps de céphaline activé (TCA) selon les protocoles suivants :

### Mesure du temps de Quick (TP)

Le temps de Quick est mesuré par l'intermédiaire du kit Neoplastine Cl (ref: 00605; Stago, France) sur un appareil de type STAR (Stago) en suivant le programme dédié. Brièvement, le plasma citraté est utilisé pur. La Néoplastine® (thromboplastine préparée à partir de tissu cérébral frais de lapin) est mélangé à la solution de calcium 25 mM et sert d'initiateur de la coagulation. Après une incubation à 37°C le plasma et l'inducteur de la coagulation sont mélangés par le biorobot et le temps de coagulation est mesuré et comparé à une série de contrôles dédiés (STA coag control; ref: 00678; Stago).

### Mesure du temps de céphaline activé (TCA)

Le TCA est mesuré par l'intermédiaire du kit STA CK prest (ref: 00597; Stago, France) sur un appareil de type STAR (Stago) en suivant le programme dédié. Brièvement, le plasma citraté est utilisé pur. La céphaline (substitut plaquettaire préparé à partir de tissu cérébral de lapin) est mélangée à la solution de kaolin (5 mg/ml) et sert d'initiateur de la coagulation. Après une incubation à 37°C le plasma et l'inducteur de la coagulation sont mélangés par le biorobot et le temps de coagulation est mesuré et comparé à une série de contrôles dédiés (STA coag control; ref: 00679; Stago).

### Résultats :

Les résultats sont illustrés dans la figure 3.

Les fractions non diluées coagulent comme le plasma de départ. Le passage sur la chromatographie d'immunoaffinité n'affecte pas la capacité du plasma à coaguler (TP et TCA) sauf sur les fractions (FNR3-4) qui sont diluées par le tampon contenu dans le volume mort de la colonne.

### Exemple 3: Préparation du plasma universel à partir d'individus donneurs humains appartenant au groupe sanguin O à l'aide de déplétion en batch

La collecte des plasmas unitaires non universels est effectuée selon la méthode décrite dans l'exemple 2.

Le mélange des plasmas unitaires non universels est obtenu selon la méthode décrite dans l'exemple 2.

L'élimination des anticorps anti-A et anti-B présents dans le mélange des plasmas unitaires non universels est effectuée par la déplétion en batch selon la méthode suivante :
- prélever 4mL de gel Iso A HyperCel® et 4mL de gel Iso B HyperCel® et mélanger les 2 gels par agitation douce
- prélever 5,5mL du mélange de gel obtenu ci-dessus pour la capture en batch
- laver le mélange de gel avec 5CV (column volume) de solution de NaCl 2M,
- laver le mélange de gel avec 4 CV de solution de phosphate 0,1M à pH8
- laver le mélange de gel avec 10CV d'eau ultrapure,
- équilibrer le mélange de gel avec 5CV de PBS filtré par un filtre de 0,22µm,
- ajouter 20mL de plasma pool dans le mélange de gel et placer sous agitation à température ambiante
- prélever respectivement 1mL du mélange gel-plasma après 10 min, 30 min, 1h, 2h d'incubation, soit 4 fractions
- récupérer le plasma à partir de ces fractions par centrifugation à 2300g pendant 5min.

### Tests de dosage :

La quantité de protéines totales présentes dans le plasma récupéré après la déplétion anti-A/anti-B en batch est mesurée.

Le résultat, illustré dans la figure 4, montre que la quantité de protéines totales n'est que légèrement baissée après la déplétion anti-A/anti-B. Cette baisse n'est pas influencée par le temps d'incubation avec le gel A HyperCel® et le gel B HyperCel®.

La quantité d'anticorps anti-A et anti-B présents dans les prélèvements obtenus après respectivement 10 min, 30 min, 60 min et 120 min d'incubation en batch a été dosée. Le résultat de dosage est donné dans le tableau 5 ci-après.

**Tableau 5**

| Prélèvement | 10 min d'incubation | 30 min d'incubation | 60 min d'incubation | 120 min d'incubation |
|---|---|---|---|---|
| % IgG 100%=plasma départ | <1,55 | <1,55 | <1,55 | <1,55 |
| % IgM 100%=plasma départ | 10 | 7 | 6 | 5 |

Ces résultats montrent que les anticorps anti-A et anti-B de type IgG et de type IgM sont éliminés majoritairement après 10 min d'incubation en batch.

### Teneur en facteurs de la coagulation présents dans le plasma universel

L'activité du facteur VIII et celle du facteur V contenus dans le plasma universel résultant de l'étape de déplétion en batch ont été testées. Les résultats sont présentés ci-après.

Le facteur V(FV) et le facteur VIII (FVIII) ont été dosés selon les protocoles de dosage décrits dans l'exemple 1.

Les résultats sont illustrés dans les figures 5A et 5B. La déplétion anti-A/anti-B en batch n'affecte pas les taux en facteur V et en facteur VIII du plasma résultant de l'étape de déplétion en batch.

Le plasma universel obtenu présente un taux de facteur V supérieur à 5µg/mL et un taux de facteur VIII supérieur à 0,6 UI/mL.

Ceci est par ailleurs confirmé par les mesures de temps de Quick et de temps de céphaline activé (TCA) selon les protocoles décrits dans l'exemple 2.

Les résultats sont illustrés dans la figure 6. Ces résultats montrent que la déplétion anti-A/anti-B en batch n'affecte pas la capacité du plasma à coaguler (TP et TCA).

### Exemple 4: Lyophilisation du plasma universel

Le plasma universel issu de l'étape de chromatographie d'immunoaffinité est réparti dans des flacons de 500 mL "de type I", de telle sorte que chacun des flacons contienne 215 mL de plasma universel.

La lyophilisation des plasmas universels contenus dans chacun des flacons précédemment obtenus se fait dans un lyophilisateur de type SMH 615, commercialisé par USIFROID. Chaque flacon est placé sur une étagère. La lyophilisation se fait dans les conditions particulières détaillées ci-après.

### A/ Pré-refroidissement

Cette étape permet le refroidissement des étagères du lyophilisateur à une température de -5°C. Cette étape permet d'éviter la dégradation des facteurs de coagulation qui sont thermosensibles pendant le temps de la répartition. Les lots sont chargés au fur et à mesure dans le lyophilisateur.

### B/ Congélation

Le plasma universel est congelé à une température de -50 °C. On maintient le produit à cette température pendant 240 minutes. La durée de la rampe est de 30 minutes et le palier est de 300 minutes.

### C/ Mise sous vide

Pour permettre la sublimation, on procédé à une mise sous vide du lyophilisateur. Le vide s'effectue pendant 2 minutes à une pression de 600 mBar (soit 0,6x10⁵ Pa).

### D/ Sublimation

Cette étape s'effectue à une température comprise entre 10 et 15°C et à une pression inférieure à 300 µBar (soit 30Pa).

Le premier palier à 10 °C de température a une rampe de 60 minutes et un palier de 3000 minutes.

Le second palier avec une température de 15 °C a une rampe de 10 minutes et un palier de 1200 minutes.

### E/ Dessiccation secondaire

Cette étape s'effectue à une température comprise entre 30 et 35°C sous une pression de 30 µBar (soit 3Pa).

Le premier palier à 35 °C a une rampe de 600 minutes et un palier de 1200 minutes. Le second palier à 30 °C a une rampe de 480 minutes et un palier de 1800 minutes.

### F/ Contrôles de la qualité du lyophilisat

Ce protocole permet l'obtention d'un plasma lyophilisé présentant un taux d'humidité relative inférieure à 2%.

### Exemple 5: Reconstitution du plasma universel

On prend un flacon de 500 mL de plasma universel. On ajoute 200 mL d'eau pour préparation injectable. On obtient ainsi un plasma universel reconstitué.

Après reconstitution, le produit obtenu doit répondre aux exigences suivantes:
- temps de reconstitution inférieur à 6 minutes ; concentration en facteur VIII supérieure ou égale à 0,5 UI/L ;
- titre en agglutinines anti A et anti B inférieur à 64;

La composition et les caractéristiques du plasma universel reconstitué sont détaillées dans le tableau 6 ci-après :

**Tableau 6 : Compositions et caractéristiques du plasma reconstitué**

| Paramètres | Unités | Plasma universel reconstitué |
|---|---|---|
| Fibrinogène | g/L | 2,4 |
| Facteur V | UI/mL | 0,7 |
| Facteur VIII | UI/mL | 0,7 |
| Facteur XI | UI/mL | 0,7 |
| Protéine C | UI/mL | 0,9 |
| Protéine S | UI/mL | 0,9 |
| Antithrombine III | UI/mL | 1,0 |
| A2 antiplasmine | UI/mL | 0,9 |
| Titre anti-A | | <1/64 |
| Titre anti-B | | <1/64 |
| Temps de reconstitution | min | <6min |

Les critères mesurés sont conformes aux exigences réglementaires. Le plasma obtenu selon le procédé de l'invention est donc adapté à une utilisation thérapeutique.

## Revendications

1. Procédé de préparation d'un plasma universel atomisé, comprenant les étapes suivantes :
a) Mélange de plasmas unitaires non universels obtenus à partir d'un échantillon d'individus donneurs,
b) Elimination simultanée des anticorps anti-A et anti-B présents dans le plasma par chromatographie d'immunoaffinité ou par déplétion en batch, et
c) Atomisation du plasma universel issu de l'étape b).

2. Procédé de préparation d'un plasma universel atomisé selon la revendication 1, dans lequel l'étape a) est réalisée sans aucune sélection préalable de donneurs.

3. Procédé de préparation d'un plasma universel atomisé selon la revendication 1, dans lequel l'étape b) est réalisée au moyen d'une chromatographie d'immunoaffinité ou par déplétion en batch sur un support greffé de groupes oligosaccharidiques antigéniquement similaires aux groupes sanguins A et B.

4. Procédé de préparation d'un plasma universel atomisé selon la revendication 3, dans lequel les groupes oligosaccharidiques représentent des trisaccharides correspondants aux épitopes des groupes sanguins A et B.

5. Procédé de préparation d'un plasma universel atomisé selon la revendication 4 dans lequel les trissacharides, correspondant à l'épitope du groupe sanguin A, présentent la structure N-acétylgalactosamine (GalNAc) - Galactose (Gal) - Fucose (Fuc) et ceux correspondant à l'épitope du groupe sanguin B, présentent la structure Galactose-Galactose-Fucose.

6. Procédé de préparation d'un plasma universel atomisé selon l'une des revendications 1 à 5, dans lequel lesdits individus donneurs appartiennent au groupe sanguin sélectionné par le groupe sanguin A, le groupe sanguin B, le groupe sanguin AB et/ou le groupe sanguin O.

7. Procédé de préparation d'un plasma universel atomisé selon l'une des revendications 1 à 5, dans lequel lesdits individus donneurs appartiennent au groupe sanguin A, au groupe sanguin B, au groupe sanguin AB et au groupe sanguin O.

8. Procédé de préparation d'un plasma universel atomisé selon l'une des revendications 1 à 5, dans lequel le plasma universel issu de l'étape b) présente une teneur en anticorps anti-A et en anticorps anti-B contenus dans le plasma universel telle que le résultat du test de Coombs est négatif à la dilution 1/64.

9. Procédé de préparation d'un plasma universel atomisé selon l'une des revendications 1 à 8, comprenant en outre une étape de sécurisation biologique.

10. Procédé de préparation d'un plasma universel atomisé selon la revendication 9, dans lequel l'étape de sécurisation biologique est réalisée par inactivation virale au moyen d'un solvant/détergent, de préférence en utilisant un solvant/détergent dialysable ou filtrable.

11. Procédé de préparation d'un plasma universel atomisé selon l'une des revendications 1 à 10, comprenant en outre une étape d'ultrafiltration.

12. Procédé de préparation d'un plasma universel atomisé selon la revendication 11, **caractérisé en ce que** l'étape d'ultrafiltration est associée à une étape de dialyse.
